# EUROPEAN PATENT APPLICATION

(11) **EP 4 283 281 A1**
(43) Date of publication of application: **29.11.2023**
(21) Application number: 22742301.9
(22) Date of filing: 17.02.2022
(51) Int. Cl.: G01N 21/47, G01R 33/07, F21S 8/00

(54) **ANALYZER AND METHOD FOR READING DETECTION RESULT OF TEST APPARATUS**

(30) Priority: 23.01.2021 CN 202120184758 U; 31.08.2021 CN 202111012904
(71) Applicant: Leadway (HK) Limited, Hong Kong (CN)
(72) Inventor: FANG, Wei, hangzhou, Zhejiang 310030 (CN); TANG, Linyong, hangzhou, Zhejiang 310030 (CN); LIU, Shengqiang, hangzhou, Zhejiang 310030 (CN); WEI, Yongchao, hangzhou, Zhejiang 310030 (CN); SHANG, Tao, hangzhou, Zhejiang 310030 (CN)
(74) Representative: Schiweck Weinzierl Koch Patentanwälte Partnerschaft mbB
(86) International application number: PCT/CN2022/076650
(87) International publication number: WO 2022/156824

(57) **Abstract**

An analyzer and a method for reading a test result of a test apparatus are provided. The analyzer includes an analyzer (1) housing, and a drive system, a photoelectric system, a data processing system and a result output system disposed in the analyzer (1) housing. The test apparatus is carried by an objective stage (10) of the drive system to get in and out of the analyzer (1) to read the test result. The analyzer (1) compact in size and stable in operation is applicable to testing of various biochemical indicators and can improve the efficiency of medical testing.

## Description

### Field of the Invention

The present invention relates to the field of medical examination, in particular to an analyzer for medical testing and a method for reading a test result of an immunoassay device.

### Background of the Invention

In vitro diagnosis is an important constituent part in the field of medical examination, which utilizes ex vivo samples to analyze various physiological indicators of subjects. For example, an immunochromatographic testing product, which can perform point-of-care testing (POCT), uses an ex vivo sample such as blood, saliva and respiratory mucus to complete testing of various physiological indicators of a subject based on immunoreaction and chromatography techniques. Now it has been widely used in the field of medical examination. The immunochromatographic testing product can obtain the test result with naked eyes or an analyzer.

An analyzer used for an immunochromatographic testing product typically includes an objective stage that stores the immunochromatographic testing product, an electric electrical machinery that transports the objective stage to the interior of the analyzer, an optical system that contains a detection light source and a photodiode, a data processing system, and a result output system. The immunochromatographic testing product is usually available in the type of a test strip, a test device, etc. As for the test device, the test strip is mounted in the test device which includes an upper cover and a bottom plate. The upper cover is provided with a sample addition hole corresponding to the sample addition area of the test strip, and provided with an observation window corresponding to the result display area of the test strip.

When testing, the test device is placed on the objective stage of the analyzer and transported to the interior of the analyzer; the observation window of the test device is located under the optical system of the analyzer; the detection light source irradiates to the result display area of the test strip through the observation window; the photodiode receives a reflected light signal of the test strip and transmits data to the data processing system to complete the analysis of the test result; and the output system outputs the test result. When the analyzer is utilized to read the test result, it needs to place the test device on the objective stage accurately to ensure that the detection light source can irradiate to the result display area of the test strip, so the test device needs to be put in place, and the upper cover and bottom plate of the test device placed on the objective stage cannot be placed reversely. However, the current analyzer is incapable of well avoiding the problems that the test device is not put in place and the test plate is placed reversely.

### Summary of the Invention

A first objective of the present invention is to provide an analyzer, comprising a drive system for transporting a test device, the drive system comprises an objective stage and an electric motor under the drive of the electric motor, the objective stage and the electrical machinery work together to make the objective stage to reciprocate in the analyzer, a photoelectric system is disposed on the operating line of the objective stage, and the photoelectric system comprises a detection light source and a light signal sensor; the analyzer is provided with a magnetic sensor chip, and a magnet steel paired with the magnetic sensor chip for use is disposed on the objective stage; and when the magnetic sensor chip receives a preset magnet steel signal value, or when the magnet steel is located under a magnetic sensor chip, the position of the objective stage is set as the starting point for the analyzer to start to calculate the number of moving steps of the objective stage.

Further, the result display area of the test strip placed on the objective stage is further away from an insertion port for the test device of the analyzer than the magnet steel on the objective stage.

Further, the objective stage is provided with a test device storage area and a magnet steel storage area, the magnet steel is located in the magnet storage area, and the magnet storage area is further away from the insertion port for the test device of the analyzer than the test device storage area when the objective stage is located in the analyzer.

Further, the analyzer further comprises a light source angle positioning plate, and the magnetic sensor chip is mounted on the light source angle positioning plate.

Further, the light source angle positioning plate is provided with a light receiving channel and a light irradiation channel.

Further, when the number of moving steps of the objective stage reaches a preset number of steps, the rear end of the result display area of the test strip located on the objective stage has traveled past the light receiving channel of the light source angle positioning plate, or the rear end of the result display area is located just under the light receiving channel.

Further, the test strip is located within the test device, the result display area of the test strip is located under the observation window of the test device, and the test device is located on the objective stage.

Further, the analyzer may further comprise a two-dimensional code scanning system. Further, a mirror is mounted on a frame above an objective stage operating channel, with the mirror surface facing the objective stage at a certain angle.

Further, a two-dimensional code scanner is provided on a side of the objective stage operating channel, and the graphic information of the two-dimensional code on the test device is captured by the two-dimensional code scanner after being mapped to the mirror.

A second objective of the present invention is to provide a method for reading the test result of a test device, which comprises: providing an analyzer; the analyzer comprises a drive system for transporting a test device and a photoelectric system, the drive system comprises an objective stage and an electric motor under the drive of the electric motor, the objective stage and the electric motor work together to make the objective stage to reciprocate in the analyzer; the analyzer is provided with a magnetic sensor chip, and a magnet steel paired with the magnetic sensor chip for use is disposed on the objective stage; when the objective stage is in a position of waiting for a user to put the test apparatus, the position of the objective stage is set as the starting point for the analyzer to start to calculate the number of moving steps of the objective stage; after the test apparatus is put into the objective stage, the electric motor drives the objective stag to operate towards the interior of the analyzer and starts to calculate the number of moving steps of the objective stage; and when the number of steps of the objective stage reaches the preset number of steps, the analyzer starts to read the test result of the test apparatus.

Further, when the objective stage is in a position of waiting for a user to put the test apparatus, the magnetic sensor chip receives a preset magnet steel signal value, or the magnet steel is located under the magnetic sensor chip.

Further, after the test apparatus is placed on the objective stage, the objective stage carries the result display area of the test apparatus to move toward the detection light source, and at the same time, the analyzer starts to record the number of moving steps of the objective stage.

Further, after the number of steps of the objective stage reaches the preset number of steps, the electric motor reverses and drives the objective stage to move toward the outside of the analyzer, and the photoelectric system starts to scan the result display area of the test apparatus.

Further, when the magnet steel on the objective stage moves to be under the magnetic sensor chip, the data processing system of the analyzer receives a magnetic induction signal and the electric motor stops reversing.

Further, the data processing system of the analyzer processes scanning information and outputs the processed result through the result output system of the analyzer. Further, the analyzer further comprises a light source angle positioning plate, and the magnetic sensor chip is mounted on the light source angle positioning plate.

Further, the light source angle positioning plate is provided with a light receiving channel and a light irradiation channel.

Further, when the number of steps of the objective stage reaches the preset number of steps, the rear end of the result display area of the test apparatus has passed through the light receiving channel of the light source angle positioning plate, or the rear end of the result display area is located just under the light receiving channel.

Further, the test apparatus comprises the test device or the test strip, and the result display area is located on the test strip.

Further, the test strip is located within the test device, and the result display area of the test strip is located under the observation window of the test device.

Further, the test apparatus comprises an immunochromatographic test device made using latex or gold label as a label, a fluorescent immunochromatographic test device labeled by fluorescent microspheres, an HCG test strip, and a urinalysis test strip.

A third objective of the present invention is to provide a light source angle positioning plate, comprising a light receiving channel and light irradiation channels; the first light irradiation channel and the second light irradiation channel are disposed around the light source angle positioning plate, the light receiving channel is located in the center position between the first light irradiation channel and the second light irradiation channel; and the angle that the light source irradiates to the horizontal plane through the first light irradiation channel is different from the angle that the light source irradiates to the horizontal plane through the second light irradiation channel.

Further, at least two first light irradiation channels and two second light irradiation channels are comprised, the two first light irradiation channels are disposed opposite each other with the light receiving channel as the center, and the two second light irradiation channels are disposed opposite each other with the light receiving channel as the center.

Further, the light path included angle between the two first light irradiation channels is different from the light path included angle between the two second light irradiation channels.

Further, the light path included angle between the two first light irradiation channels is 70°, and the light path included angle between the two second light irradiation channels is 90°.

Further, an LED lamp is mounted in the first light irradiation channel, and a fluorescent lamp is mounted in the second light irradiation channel.

Further, the light source angle positioning plate is six petal-shaped, the center position of the light source angle positioning plate is perpendicularly provided with a light receiving channel, and two opposing first light irradiation channels, two opposing second light irradiation channels and two opposing third light irradiation channels are evenly disposed around the light receiving channel.

A fourth objective of the present invention is to provide an objective stage, comprising a test device clamping slot and a bottom plate; and an elastic component is disposed on the bottom plate, and is compressible by the test device toward the direction of the bottom plate.

Further, the bottom plate is provided with a mounting hole for the elastic component.

Further, the structure of the elastic component is an arch-shaped elastic sheet, the two ends of the arch-shaped elastic sheet are buried in the mounting hole, and the middle projection of the arch-shaped elastic sheet protrudes out of the upper surface of the bottom plate.

Further, at least one end of the arch-shaped elastic sheet is fixedly connected to the inner wall of the mounting hole by a fixing member.

Further, one end of the elastic sheet rests against the inner wall of the mounting hole without the fixing member, and the other end of the elastic sheet is fixedly connected in the mounting hole.

Further, the mounting hole for the elastic component is located on the centerline of the bottom plate.

The present invention has the following beneficial effects:
The mounting way of the elastic component on the objective stage of the analyzer can achieve the fact that the test device also can be smoothly extracted from the objective stage when the test device is placed in a wrong direction.

The light source angle positioning plate of the analyzer is ingenious in structure, can meet the needs of different angles of light irradiation, can provide a variety of light sources to apply to the needs of test strips with a variety of different testing principles, and is simple and convenient to use.

The magnetic sensor chip cooperates with the corresponding magnet steel to precisely control the movement of the objective stage in the analyzer and accurately transport the test device to an analysis position to ensure that the test result of the test strip can be precisely scanned and ensure the accuracy of the test result. Moreover, the product structure of the analyzer is combined with the usage method, and the analyzer reversely moves the objective stage to the outside of the analyzer by transporting the objective stage to the interior of the analyzer first and positioning it, in order to ensure that the result display area is fully away from light to ensure the accuracy of scanning. The two-dimensional code scanner with relatively large size is disposed on a side of the objective stage, the mirror with relatively small size is disposed above the objective stage, and such a structure design can reduce the overall height of the analyzer.

The analyzer compact in size, stable in operation and strong in practicality is applicable to testing of a variety of biochemical indicators and can improve the efficiency of medical testing.

### Brief Description of the Drawings

Fig. 1a is a schematic diagram of the internal structure of an analyzer.
Fig. 1b is a schematic diagram of the internal structure of the analyzer shown in Fig. 1a from another perspective.
Fig. 1c is a schematic diagram of the internal structure of the analyzer shown in Fig. 1a from still another perspective.
Fig. 1d is a schematic diagram of the bottom of the internal structure of the analyzer shown in Fig. 1a.
Fig. 2 is a schematic diagram of a test device being placed on an objective stage.
Fig. 3 is a schematic diagram of the objective stage.
Fig. 4 is a front view of the objective stage shown in Fig. 3.
Fig. 5 is a sectional view of Fig. 4 in the direction of A-A.
Fig. 6 is a schematic diagram of a light source angle positioning plate.
Fig. 7 is a schematic diagram of the bottom of the light source angle positioning plate shown in Fig. 6.
Fig. 8 is a schematic diagram of the top of the light source angle positioning plate shown in Fig. 6.
Fig. 8a is a sectional view of Fig. 8 in the direction of A-A.
Fig. 8b is a sectional view of Fig. 8 in the direction of B-B.
Fig. 9a is a schematic diagram of a light source being inserted into the light source angle positioning plate.
Fig. 9b is a schematic diagram of the bottom of Fig. 9a.
Fig. 10a is a schematic diagram of the light source angle positioning plate being mounted on a photoelectric panel.
Fig. 10b is a schematic diagram of the light source angle positioning plate being mounted on the photoelectric panel from another perspective.
Fig. 10c is a schematic diagram of the light source angle positioning plate being mounted on the photoelectric panel from still another perspective.
Fig. 11 is a schematic diagram of the objective stage operating to extend out of the analyzer.
Fig. 11a is a partially enlarged view of Fig. 11.
Fig. 12 is a schematic diagram of a test device being placed on the objective stage.
Fig. 13 is a schematic diagram of the objective stage with the test device entering the interior of the analyzer.
Fig. 14 is a diagram of a light path for capturing two-dimensional code information.
Fig. 15 is a schematic diagram of an in vitro diagnosis analyzer.

### Detailed Description of the Embodiments

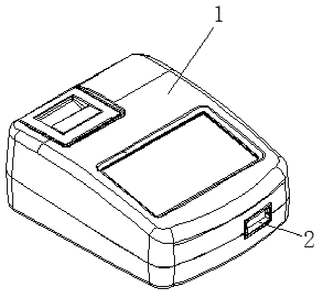

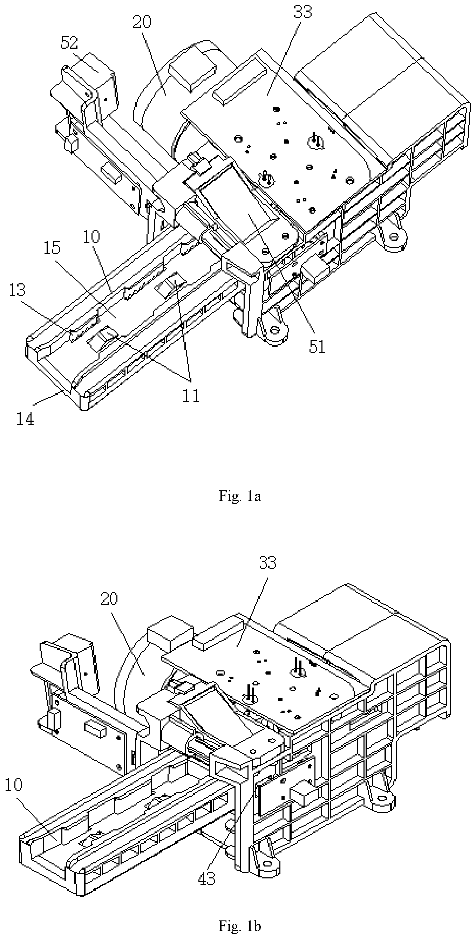

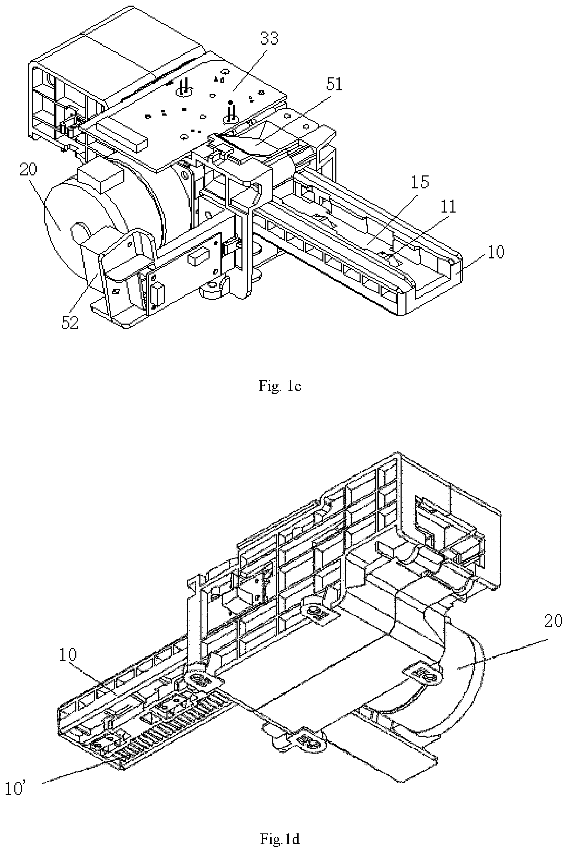

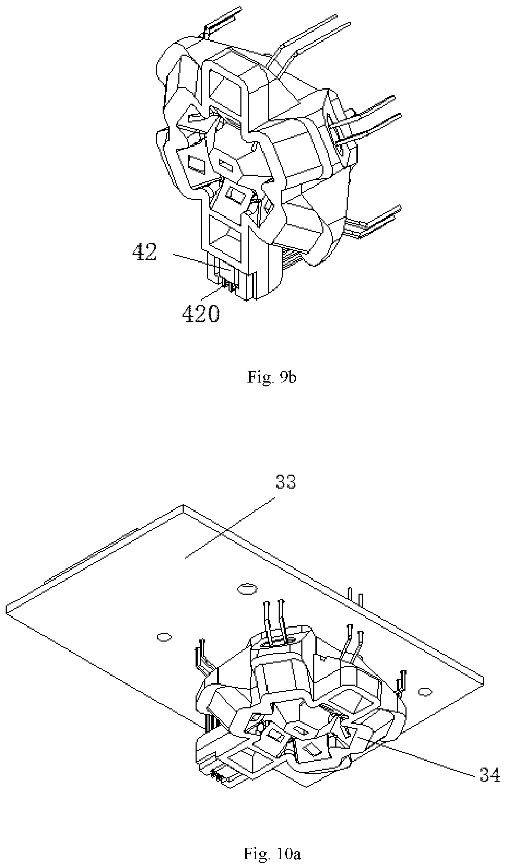

An in vitro diagnosis analyzer 1, as described in Figs. 1a to Figs. 1d and Figs. 2 to 15, includes an analyzer housing, and a drive system, a photoelectric system, a data processing system and a result output system disposed within the analyzer housing. The drive system includes an objective stage and an electric motor. The photoelectric system includes a detection light source, a light signal sensor, and a photoelectric panel. Wherein the photoelectric system is disposed on the operating line of the objective stage.

The objective stage 10 cooperates with the electric motor 20 in linkage, and the objective stage reciprocates in the analyzer under the drive of the electric motor. The electric motor starts, the test device on the objective stage is moved to the detection light source with the rotation of the electric motor, and the analyzer completes the testing. For example, the bottom of the objective stage 10 is provided with a rack 10', which cooperates with the gear on the electric motor to realize the movement of the objective stage.

As shown in Figs. 1a to Figs. 1d and Figs. 2 to 5, the objective stage is provided with a test device storage area 15 enclosed by test device clamping slots 13 on both sides and the bottom plate 14 of the objective stage, and the test device 100 is inserted into the test device storage area 15 along the test device clamping slot.

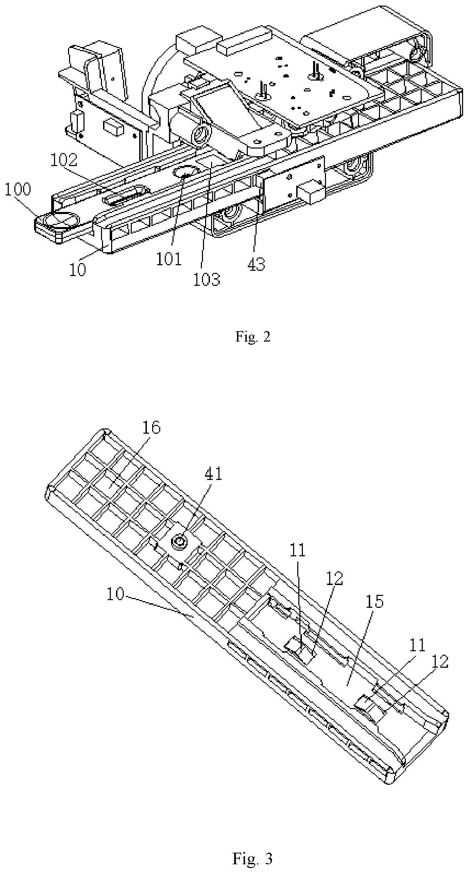

As shown in Figs. 3 to Figs. 5, elastic components are disposed on the bottom plate 14, and the elastic components are compressible by the test device 100 toward the direction of the bottom plate 14. In one embodiment, the structure of the elastic component is an arch-shaped elastic sheet 11. Mounting holes 12 are formed in the bottom plate 14 of the objective stage 10, and the arch-shaped elastic sheets 11 are mounted in the mounting holes 12. Both ends of the arch-shaped elastic sheet 11 are buried in the mounting hole 12, and a middle projection 113 of the arch-shaped elastic sheet 11 protrudes out of the upper surface of the bottom plate 14. In a preferred embodiment, the elastic components and the mounting holes are located on the centerline of the bottom plate.

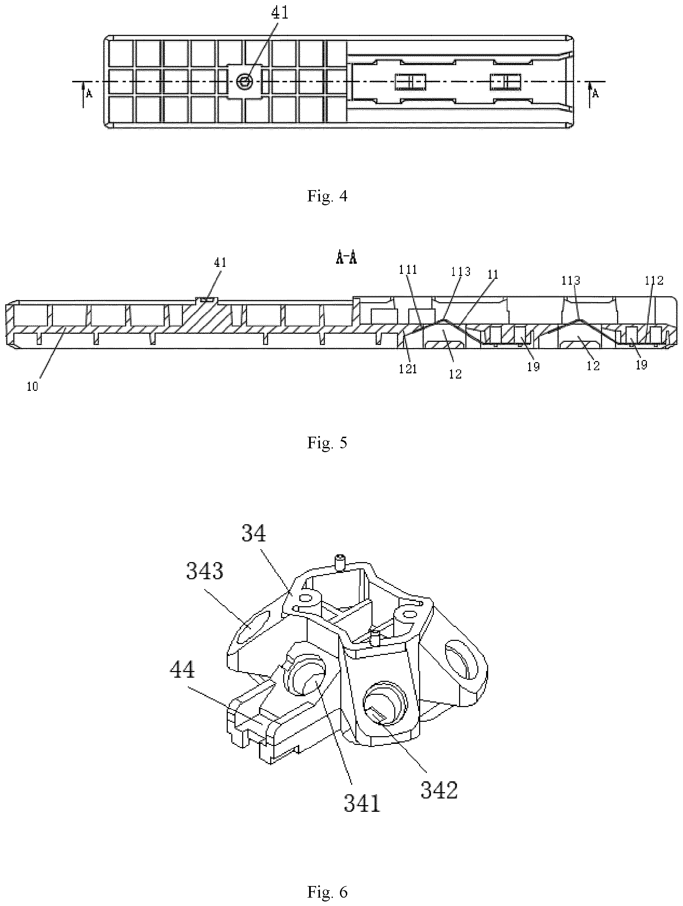

In a more specific design, as shown in Fig. 5, both ends 111 and 112 of the elastic sheet are buried in the mounting hole 12, one end 111 of the elastic sheet directly rests against the inner wall 121 of the mounting hole and the other end 112 of the elastic sheet is fixed in the mounting hole with a screw 19, and both ends of the elastic sheet will not be cocked to expose to the upper surface of the bottom plate 14 of the objective stage. The middle projection 113 of the arc-shaped elastic sheet 11 protrudes out of the upper surface of the bottom plate 14. In an additional embodiment, both ends of the elastic sheet can be fixed to the inner wall of the mounting hole with screws.

In the example shown in Fig. 1a and Fig. 2, in the process of inserting the test device into the test device storage area along the test device clamping slot, the front end of the test device squeezes the elastic sheet down along the bevel of the arc-shaped elastic sheet, the bottom plate of the test device comes into contact with the arc-shaped elastic sheet, and the rebound force of the arc-shaped elastic sheet squeezes the test device on the upper edge of the test device clamping slot 13, so that the test device 100 is more stably fastened in the test device storage area 15. Also because the arc-shaped elastic sheets are disposed on the objective stage, the test device clamping slot 13 of the objective stage or the test device 100 can have a greater machining error within an allowable range.

In the present invention, the objective stage 10 is used to place the testapparatus. In some embodiments, the test apparatus includes the test device and a test strip. That is, either the test device loaded with the test strip or a separate test strip can be placed on the objective stage.

The upper cover of the test device 100 is provided with a sample addition hole 101 and an observation window 102.

If one end of the arc-shaped elastic sheet 11 is cocked above the mounting hole, when the upper board of the test device 100 is incorrectly inserted toward the bottom plate 14 of the objective stage, the end of the elastic sheet will be easily inserted into the sample addition hole or the observation window to jam the test device. On the contrary, if the two ends 111 and 112 of the arc-shaped elastic sheet are buried in the mounting hole, when the upper board of the test device 100 is incorrectly inserted toward the bottom plate 14 of the objective stage, the arc-shaped elastic sheet will not be inserted into the sample addition hole or the observation window. It ensures that the test device placed reversely can be removed smoothly and repositioned.

The arc-shaped elastic sheet as an elastic component has a material that can be selected from a plastic sheet, a metal sheet such as a copper sheet, or an elastic ball, or the like.

The test strip 200 is mounted in the test device 100, and the test strip includes a sample receiving area 201 and a result display area 202, wherein the front end 203 of the result display area is close to the sample receiving area 201 and the rear end 204 of the result display area is far away from the sample receiving area 201. The sample receiving area 201 of the test strip is located under the sample addition hole 101, and the result display area 202 is located under the observation window 102.

To ensure that the test device placed on the objective stage has been put in position, the analyzer 1 is provided with an optocoupler switch. In one embodiment, the light path of the optocoupler switch is disposed at a position where the test device has been put in place: if the test device has been put in place, the light path of the optocoupler switch is blocked out by the test device, and if the test device is not put in place and has a certain distance from the accurate position, the light path of the optocoupler switch is on. The analyzer determines whether the test device has been placed correctly based on the information that the light path detected by the optocoupler switch is on or off. If the test device is not put in position, the analyzer will not start the testing. The optocoupler switch can be a test device sensing module 43, and also can be replaced with other sensing switch.

As shown in Figs. 6 to 10c, detection light sources 31 and the light signal sensor 32 of the analyzer 1 are electrically connected with the photoelectric panel 33, respectively. Said detection light source 31 can be selected from a light emitting diode (LED) or a fluorescent emitter, etc., and said light signal sensor can be selected from a photodiode (PD), etc. The detection light source and the light signal sensor are welded to the photoelectric panel through pins. Specifically, the light emitted from the detection light source irradiates to the result display area of the test strip, the light signal sensor obtains a light signal from the result display area and converts the light signal into an electrical signal, and the electrical signal is transmitted to the data processing system for processing. For test strips with different detection principles, different light sources can be used. For example, for the test device made using latex or gold label as a label, an LED light source 311 can be used; and for the test device labeled by fluorescent microspheres, a fluorescent light source 312 can be used. In one specific embodiment, detection light sources such as LEDs equidistantly surround the PD.

As shown in Figs. 6 to Figs. 9b, the analyzer may also include a light source angle positioning plate 34 for controlling the incident angles of the detection light sources. In the embodiment as shown in the figure, the light source angle positioning plate 34 is six-petal shaped. A light receiving channel 340 is perpendicularly disposed at the center position of the light source angle positioning plate 34, and the light signal (for example, fluorescence, reflected light or projected light, etc.) from the result display area of the test strip is transmitted to the light signal sensor 32 through the light receiving channel 340, and the light signal sensor 32 may be mounted in the light receiving channel 340. Light irradiation channels 341, 342 and 343, etc. are disposed around the light receiving channel 340 to adjust the irradiation angles of the detection light sources, and the light emitted from the detection light sources located in the light irradiation channels 341, 342 and 343 can irradiate to a testing area and then is refracted or reflected into the light receiving channel 340 by adjustment of the light irradiation channels 341, 342 and 343.

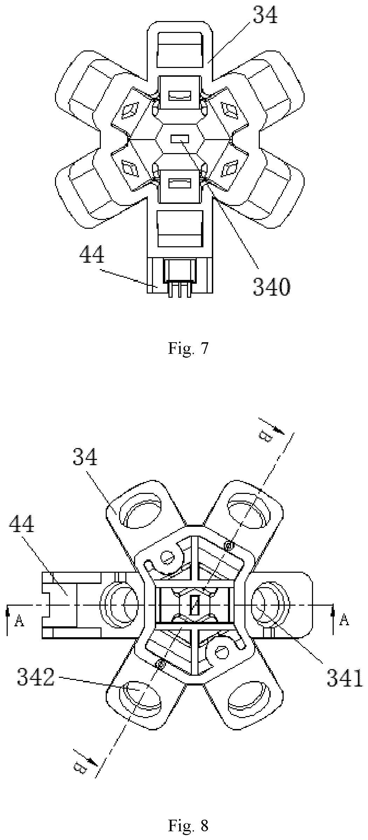

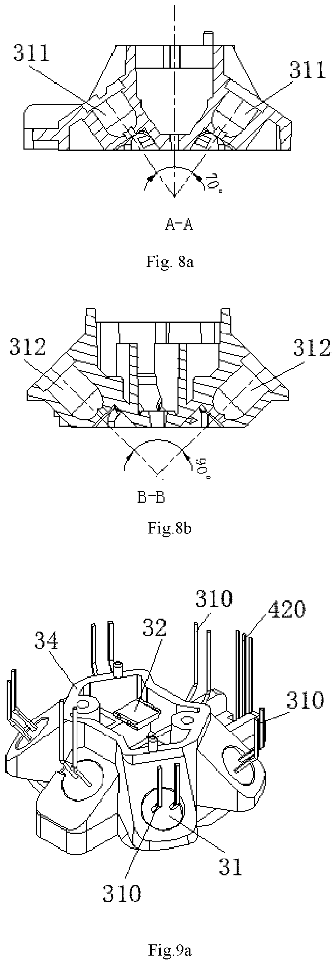

The irradiation angle can be the same or different for different light irradiation channels. As shown in Figs. 8 and 8a, two first light irradiation channels 341 disposed opposite each other have an included angle of 70°, i.e., they form an angle of 35° with the perpendicular line, respectively. As shown in Figs. 8 and 8b, the two second light irradiation channels 342 disposed opposite each other have an included angle of 90°, i.e., they form an angle of 45° with the perpendicular line, respectively. Different detection light sources can be mounted on the analyzer, for example, LED lamps 311 are mounted in the first light irradiation channels 341 shown in Fig. 8a, and fluorescent lamps 312 are mounted in the second light irradiation channels 342. When being mounted, the LED lamps or the fluorescent lamps are inserted into the angle positioning plate to form different angles of irradiation light. The pins 310 of the lamps are welded to the photoelectric panel 33.

In one embodiment, a first light irradiation channel 341 and a second light irradiation channel 342 for the adjustment of the irradiation angles of the detection light sources are disposed around the light receiving channel 340; the light receiving channel 340 is located in the center position between the first light irradiation channel 341 and the second light irradiation channel 342; and the angle that the light source irradiates to the horizontal plane through the first light irradiation channel 341 is different from the angle that the light source irradiates to the horizontal plane through the second light irradiation channel 342. The light source of the first light irradiation channel may be the same as or different from the light source of the second light irradiation channel. In another embodiment, at least two first light irradiation channels 341 and two second light irradiation channels 342 are disposed around the light receiving channel 340, the two first light irradiation channels 314 are disposed opposite each other with the light receiving channel 340 as the center, and the two second light irradiation channels 342 are disposed opposite each other with the light receiving channel 340 as the center. The two first light irradiation channels have the same light source, and the two second light irradiation channels have the same light source. Wherein the light source of the first light irradiation channel may be the same as or different from the light source of the second light irradiation channel.

In another embodiment, third light irradiation channels 343 are also disposed around the light receiving channel 340, and two opposing first light irradiation channels 341, two opposing second light irradiation channels 342 and two opposing third light irradiation channels 343 are evenly disposed around the light receiving channel 340. The light sources in the two third light irradiation channels are the same, and the light sources in the third light irradiation channels may be the same as or different from the light sources disposed in the first light irradiation channels and the light source disposed in the second light irradiation channels.

The analyzer 1 is provided with a magnetic sensor chip 42, and a magnet steel 41 corresponding to the magnetic sensor chip is disposed on the objective stage 10. The magnetic sensor chip is used to receive a magnet steel signal, and the magnetic sensor chip can be disposed on any component other than the objective stage, for example, an internal bracket of the analyzer, the light source angle positioning plate, etc. are all components other than the objective stage. In some implementations, a magnet steel signal value is preset in the magnetic sensor chip, such as a magnetic field strength greater than 75 gauss. When the magnetic sensor chip senses the preset magnet steel signal value (magnetic field strength greater than 75 gauss) in the process that the objective stage carries the magnet steel to move toward the magnetic sensor chip, the signal is transmitted to the data processing system, the analyzer receives the signal, the electric motor stops rotating, and the objective stage stops movement. At this time, the position of the objective stage is set as the starting point for the analyzer to start to calculate the number of moving steps of the objective stage.

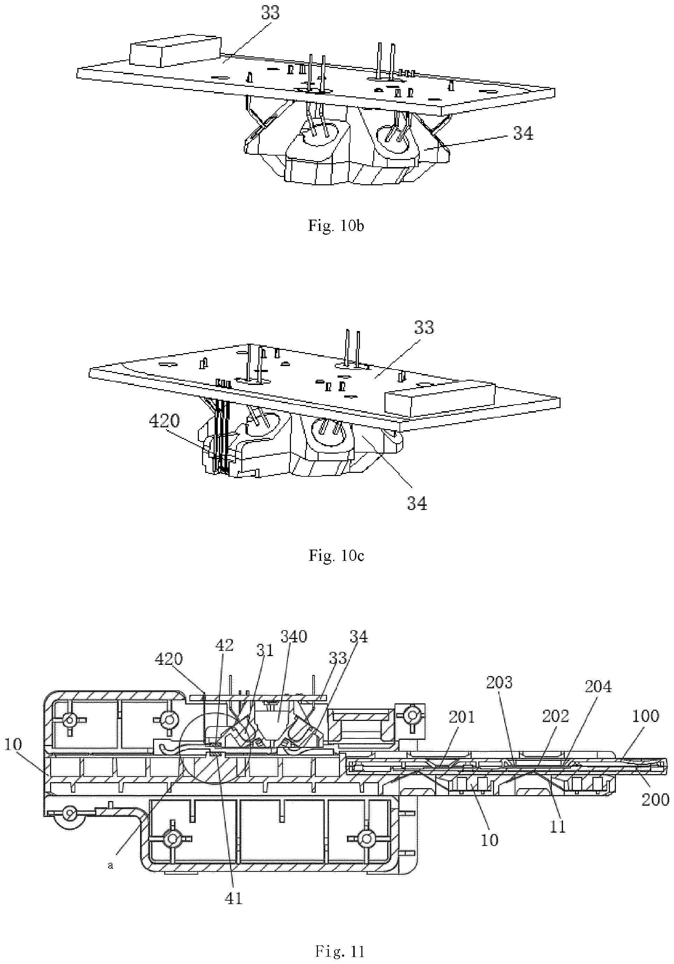

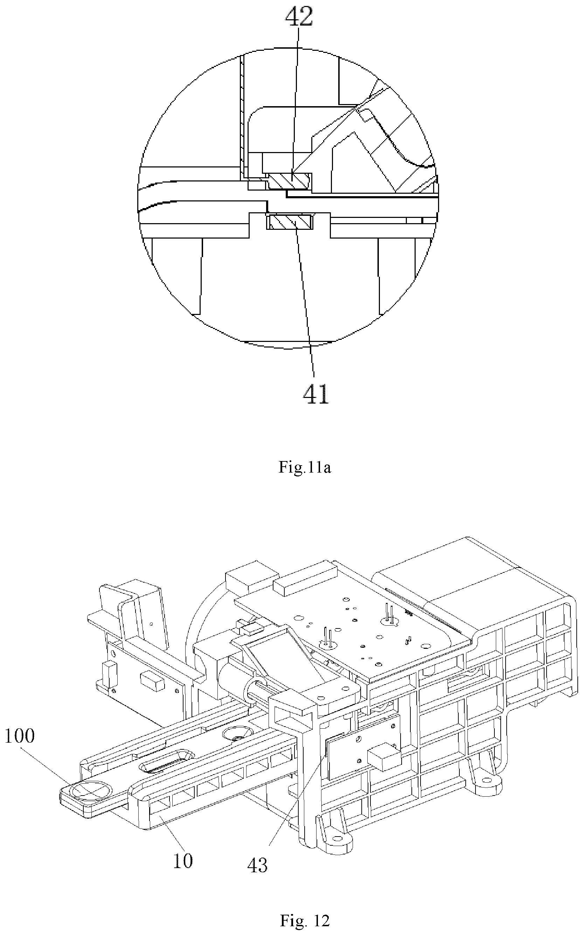

As shown in Fig. 11, when the analyzer 1 is powered on for use, the objective stage 10 will come out of the analyzer first, and the magnet steel 41 of the objective stage moves toward the position of the magnetic sensor chip 42; and when the magnetic field strength sensed by the chip is greater than 75 gauss (typical value), the sensor outputs a low level, the analyzer receives a signal, the electric motor stops rotating, and the objective stage 10 stops and keeps stationary, at which time the user can insert the test device. At this time, the position of the objective stage is also the starting point for software to start to calculate the number of moving steps of the objective stage.

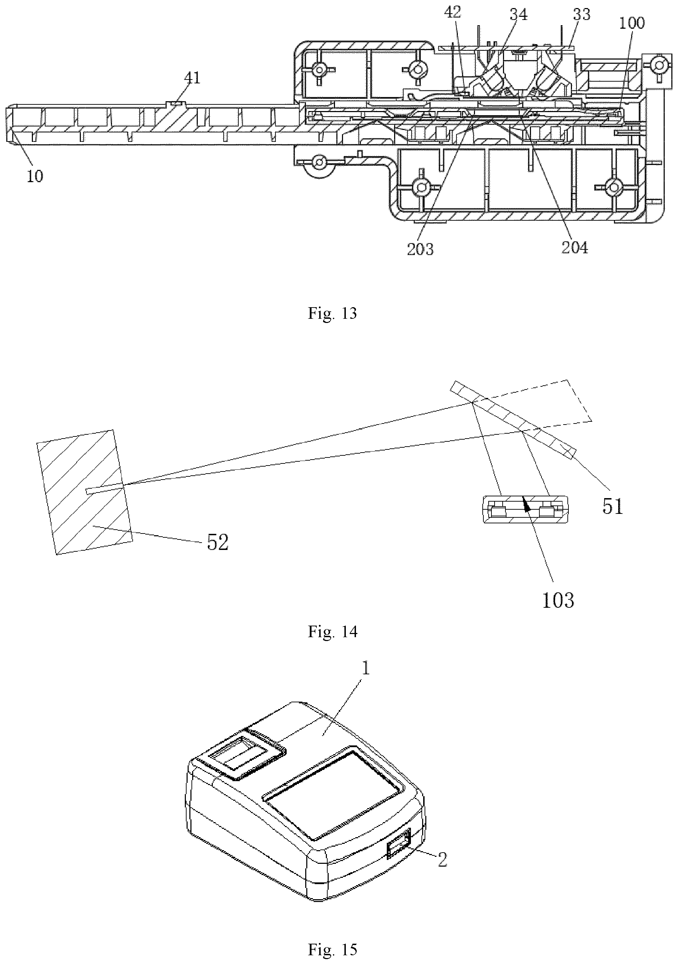

Specifically, as shown in Fig. 3, the objective stage 10 is provided with two areas, which are a test device storage area 15 and a magnet steel storage area 16, respectively; the test device storage area and the magnet steel storage area each account for about half of the objective stage in the longitudinal length; and when the objective stage is entirely in the analyzer, the test device storage area 15 is closer to the insertion port 2 for the test device of the analyzer, as shown in Fig. 13 and Fig. 15. The magnet steel 41 is mounted in the magnet steel storage area 16. As shown in Figs. 7, 9a, 9b, 10a, 10b, 10c, 11 and 13, in one embodiment, the magnetic sensor chip 42 is an in-line magnetic sensor chip, the light source angle positioning plate 34 is provided with a chip mounting plate 44, and the in-line magnetic sensor chip is mounted on the chip mounting plate 44 via pins 420. More specifically, the chip mounting plate 44 is connected to the outer wall of some one of the light irradiation channels. In one specific embodiment, as shown in Fig. 11, the chip mounting plate 44 is located on the outer wall of one light irradiation channel farthest from the insertion port 2 for the analyzer.

In addition, the present invention further provides a method for reading a test result of a test apparatus, specifically as follows:
When the objective stage is in a position in the analyzer of waiting for a user to put the test apparatus, the magnetic sensor chip receives a preset magnet steel signal value, or the magnet steel is located under the magnetic sensor chip; and the position of the objective stage is the starting point for the analyzer to start to calculate the number of moving steps of the objective stage.

When the number of steps of the objective stage reaches a preset number of steps, the rear end of the result display area of the test apparatus has passed through the light receiving channel of the light source angle positioning plate, or the rear end of the result display area is located just under the light receiving channel, at which time the electric motor reverses to drive the objective stage to move toward the outside of the analyzer, the photoelectric system is turned on while the electric motor reverses, and the turned-on photoelectric system starts to scan the result display area of the test apparatus. At this time, the test apparatus on the objective stage, that is, the test device or the result display area of the test strip passes through the light receiving channel from the rear end to the front end in sequence, the entire result display area is scanned.

When the magnet steel on the objective stage moves to be under the magnetic sensor chip on the light source angle positioning plate, the data processing system of the analyzer receives a magnetic induction signal and sends an instruction of stopping rotating to the electrical machinery, then the electrical machinery stops rotating, and the objective stage also stops movement accordingly to wait the user to remove the test apparatus from the objective stage.

After completion of the scanning, the data processing system of the analyzer processes scanning information and outputs the processed result through the result output system of the analyzer.

The analyzer may further include a two-dimensional code scanning system, a mirror is mounted on the frame above the objective stage operating channel, with the mirror surface facing the objective stage at a certain angle, and the two-dimensional code scanner is disposed at a side of the objective stage operating channel, the graphic information of the two-dimensional code on the test device is captured by the two-dimensional code scanner after being mapped to the mirror.

Wherein the test apparatus includes the test device or the test strip, and the result display area is located on the test strip; or the test device includes an immunochromatographic test device made using latex or gold label as a label, a fluorescent immunochromatographic test device labeled by fluorescent microspheres, an HCG test strip, and a urinalysis test strip.

Moreover, the test strip is located within the test device, and the result display area of the test strip is located under the observation window of the test device.

In the present invention, the analyzer reversely moves the objective stage to the outside of the analyzer after transporting the objective stage to the interior of the analyzer first and positioning it, in order to ensure that the result display area is fully away from light to ensure the accuracy of scanning. Particularly, the fluorescent test requires to be away from light, and scanning by moving the objective stage from the interior of the analyzer to the outside can reduce the influence of external light to the test result.

The test device using immunofluorescence chromatography is taken as an example to illustrate the operating process of the analyzer.

As shown in Figs. 11-14, the analyzer is powered on, and the objective stage gradually moves toward the removing/placement position of the test device under the drive of the electric motor. When the magnet steel 41 mounted on the objective stage 10 moves to be under the magnetic sensor chip 42 mounted on the light source angle positioning plate 33, i.e., the magnetic sensor chip 42 receives the preset magnet steel signal value, the data processing system of the analyzer receives a magnetic induction signal and sends an instruction of stopping rotating to the electric motor 20, then the electric motor stops rotating, and the objective stage 10 also stops and keeps stationary accordingly to wait the user to insert the test device 100 into the objective stage. At this time, the position of the objective stage is also the starting point for the data processing system to start to calculate the number of moving steps of the objective stage.

The user inserts the test device into the test device storage area 15 horizontally with the observation window side facing upward. After the test device sensing module 43 detects that the test device 100 has been accurately inserted into the objective stage 10, the test device reaches the specified position on the objective stage, the light path of the test device sensing module is cut off by the test device, the test device sensing module sends the detected signal to the data processing system, the data processing system gives an instruction of restarting to the electric motor, and the objective stage with the test device moves toward the interior of the analyzer with the rotation of the electric motor.

As shown in Fig. 13, when the number of moving steps of the objective stage recorded by the data processing system reaches the preset number of steps, the rear end 204 of the result display area of the test strip inside the test device has traveled past the light receiving channel 340 of the light source angle positioning plate, or the rear end 204 of the result display area is located just under the light receiving channel 340. At this time, the electric motor reverses (transporting the objective stage toward the outside of the analyzer) and turns on the fluorescent lamp of the photoelectric system. The objective stage moves outward with the reverse rotation of the electric motor, and the turned-on photoelectric system starts to scan the result display area of the test strip. The result display area of the test strip in the test device on the objective stage passes through the light receiving channel 340 from the rear end 204 to the front end 203 in sequence, the entire result display area is sequentially scanned. When the magnet steel 41 on the objective stage moves to be under the magnetic sensor chip on the light source angle positioning plate, the data processing system of the analyzer receives a magnetic induction signal and sends an instruction of stopping rotating to the electric motor, then the electric motor stops rotating, and the objective stage also stops and keeps stationary accordingly to wait the user to remove the test device from the objective stage.

After processing scanning information, the data processing system of the analyzer 1 outputs the processed result through the result output system of the analyzer 1.

The analyzer 1 may further include a two-dimensional code scanning system for reading the two-dimensional code information on the test device 100. The analyzer as shown in Fig. 1a and Fig. 14, has a mirror 51 mounted on a frame above the objective stage operating channel, with the mirror surface facing the objective stage at a certain angle. A two-dimensional code scanner 52 is disposed on a side of the objective stage operating channel. The two-dimensional code scanner can capture the image presented on the mirror surface. As shown in Fig. 14, the upper surface of the test device on the objective stage is provided with a two-dimensional code. When the objective stage travels to drive the two-dimensional code of the test device to fall within the irradiation range of the mirror, the graphic information of the two-dimensional code 103 on the test device is captured by the two-dimensional code scanner 52 after being mapped to the mirror 51. The angle at which the mirror is disposed on the analyzer is usually determined based on the receiving angle of the two-dimensional code module. For example, if the receiving angle of the two-dimensional code module is 15-25 degrees, the mirror is adjusted to this suitable angle to allow the two-dimensional code module to be capable of receiving the two-dimensional code presented in the mirror. The two-dimensional code scanner transmits the captured two-dimensional code information to the data processing system of the analyzer to parse out the two-dimensional code information. In one embodiment, the two-dimensional code scanner with relatively large size is disposed on a side of the objective stage, the mirror with relatively small size is disposed above the objective stage, and such a structure design can reduce the overall height of the analyzer.

The analyzer described in the present invention can be used for the testing of a dry test strip or a test device, for example, for testing of a variety of biochemical indicators using an immunochromatographic test device made with latex or gold label as a label, a fluorescent immunochromatographic test device labeled with fluorescent microspheres, an HCG test strip, a urinalysis test strip, etc. The analyzer described in the present invention is small in size and has accurate test result.

## Claims

1. An analyzer, comprising a drive system for transporting a test apparatus and a photoelectric system, wherein the drive system comprises an objective stage and an electric motor, under the drive of the electric motor, the objective stage and the electrical machinery work together to make the objective stage to reciprocate in the analyzer; and the analyzer is provided with a magnetic sensor chip, and a magnet steel paired with the magnetic sensor chip for use is disposed on the objective stage.

2. The analyzer according to claim 1, wherein the position of the objective stage while waiting for a user to put the test device is set as the starting point for the analyzer to start to calculate the number of moving steps of the objective stage.

3. The analyzer according to claim 1, wherein when the objective stage is in a position of waiting for a user to put the test device, the magnet steel is located under the magnetic sensor chip.

4. The analyzer according to claim 1, wherein the objective stage is provided with a test device storage area and a magnet steel storage area, and the test device storage area is closer to the insertion port for the test device of the analyzer when the objective stage is entirely located in the analyzer.

5. The analyzer according to claim 1, wherein the analyzer further comprises a light source angle positioning plate, and the magnetic sensor chip is mounted on the light source angle positioning plate.

6. The analyzer according to claim 5, wherein the light source angle positioning plate is provided with a light receiving channel and a light irradiation channel.

7. The analyzer according to claim 6, wherein when the number of moving steps of the objective stage reaches a preset number of steps, the rear end of the result display area of the test strip located on the objective stage has traveled past the light receiving channel of the light source angle positioning plate, or the rear end of the result display area is located just under the light receiving channel.

8. A method for reading a test result of a test apparatus, comprising: providing an analyzer, wherein the analyzer comprises a drive system for transporting a test apparatus and a photoelectric system, the drive system comprises an objective stage and a electrical machinery under the drive of the electric motor, the objective stage and the electric motor work together to make the objective stage to reciprocate in the analyzer; the analyzer is provided with a magnetic sensor chip, and a magnet steel paired with the magnetic sensor chip for use is disposed on the objective stage; when the objective stage is in a position of waiting for a user to put the test apparatus, the position of the objective stage is set as the starting point for the analyzer to start to calculate the number of moving steps of the objective stage; after the test apparatus is put into the objective stage, the electrical machinery drives the objective stag to operate towards the interior of the analyzer and starts to calculate the number of moving steps of the objective stage; and when the number of steps of the objective stage reaches the preset number of steps, the analyzer starts to read the test result of the test apparatus.

9. The method according to claim 8, wherein when the objective stage is in a position of waiting for a user to put the test apparatus, the magnetic sensor chip receives a preset magnet steel signal value, or the magnet steel is located under the magnetic sensor chip.

10. The method according to claim 8, wherein after the number of steps of the objective stage reaches the preset number of steps, the electric motor reverses and drives the objective stage to move toward the outside of the analyzer, and the photoelectric system starts to scan the result display area of the test apparatus.

11. The method according to claim 10, wherein when the magnet steel on the objective stage moves to be under the magnetic sensor chip, the data processing system of the analyzer receives a magnetic induction signal and the electric motor stops reversing.

12. The method according to claim 10, wherein the data processing system of the analyzer processes scanning information and outputs the processed result through the result output system of the analyzer.

13. The method according to claim 8, wherein the analyzer further comprises a light source angle positioning plate, and the magnetic sensor chip is mounted on the light source angle positioning plate.

14. The method according to claim 13, wherein the light source angle positioning plate is provided with a light receiving channel and a light irradiation channel.

15. The method according to claim 14, wherein when the number of steps of the objective stage reaches a preset number of steps, the rear end of the result display area of the test apparatus has passed through the light receiving channel of the light source angle positioning plate, or the rear end of the result display area is located just under the light receiving channel.
